# EUROPEAN PATENT SPECIFICATION

(11) **EP 4 085 858 B1**
(45) Date of publication and mention of the grant of the patent: **02.12.2026**
(21) Application number: 20909204.8
(22) Date of filing: 29.09.2020
(51) Int. Cl.: A61B 18/12, A61B 18/14, G01R 27/02, G01K 13/00, A61B 18/00, G01K 7/22, G01K 13/20

(54) **DETECTION MECHANISM, RADIO-FREQUENCY ABLATION CATHETER AND RADIO-FREQUENCY ABLATION SYSTEM**
DETEKTIONSMECHANISMUS, HOCHFREQUENZ-ABLATIONSKATHETER UND HOCHFREQUENZ-ABLATIONSSYSTEM
MÉCANISME DE DÉTECTION, CATHÉTER D'ABLATION PAR RADIOFRÉQUENCE ET SYSTÈME D'ABLATION PAR RADIOFRÉQUENCE

(30) Priority: 31.12.2019 CN 201911403441
(43) Date of publication of application: 09.11.2022
(73) Proprietor: Hangzhou Broncus Medical Co., Ltd., Hangzhou, Zhejiang 310051 (CN)
(72) Inventor: WANG, Liming, Hangzhou, Zhejiang 310051 (CN); XU, Hong, Hangzhou, Zhejiang 310051 (CN); ZHOU, Huazhen, Hangzhou, Zhejiang 310051 (CN)
(74) Representative: MERH-IP Matias Erny Reichl Hoffmann Patentanwälte PartG mbB
(86) International application number: PCT/CN2020/118646
(87) International publication number: WO 2021/135461

(56) References cited:
- WO-A1-03/090636
- WO-A1-2011/146243
- WO-A2-2007/005830
- CN-A- 1 147 964
- CN-A- 105 935 314
- CN-A- 110 074 860
- CN-A- 110 074 860
- CN-A- 110 960 314
- CN-A- 110 974 406
- CN-Y- 2 855 351
- US-A1- 2011 202 053
- US-B2- 8 109 926
- US-B2- 9 675 404

## Description

### TECHNICAL FIELD

The present invention relates to the technical field of medical devices, and in particular to a radio-frequency ablation catheter with a detection mechanism and a radio-frequency ablation system.

### BACKGROUND

Radio-frequency ablation technology is widely used in lung surgery. Radio frequency is not a band divided in wireless communications, and exert mainly a thermal effect in organisms. When a radio-frequency current frequency reaches a value (>100kHz), charged ions in a tissue is caused to move and heat is generated from friction (60 °C to 100°C). A frequency commonly used by a radio-frequency ablation apparatus is 200 to 500 kHz, and the output frequency is 100to 400kHz. An ablation electrode is a core component in a radio-frequency ablation system, because it directly affects the size and shape of coagulation necrosis. A coagulation area desirably has a spherical or spheroidic shape. A multi-needle electrode is guided by B ultrasound or CT to directly pierce into a pathological tissue mass of human, and the radio-frequency electrode needle causes the temperature in the tissue to rise to 60°C or higher, leading to the death of cells and the formation of a necrotic area. For example, the local temperature in the tissue exceeds 100°C, causing coagulation necrosis of the tumor tissue and parenchyma surrounding organs. During treatment, a large spherical area of coagulation necrosis can be formed, and there is also a hyperthermia area of 43-60°C outside the area of coagulation necrosis. In this area, cancer cells are killed, and normal cells can be restored.

During the treatment process, a radio-frequency electrode is inserted into a human tissue, a current is introduced to the lesion by the radio-frequency electrode, and a lot of heat is generated by the radio-frequency electrode. For example, when the temperature of the lesion reaches 40°C-60°C and is maintained for a period of time, the ablation operation of the lesion is completed. However, since the radio-frequency ablation system in the prior art cannot determine the working status information of the radio-frequency electrode, for example, the temperature near the radio-frequency electrode, the progress of the ablation operation can only be empirically determined and then an adjustment is made by a physician during surgery, which increases the difficulty and accuracy of the operation. Therefore, there is an urgent need in the art to provide a radio-frequency ablation system able to accurately determine whether the ablation is completed. United State Patent No. US9675404B2 discloses an electrosurgical system that can apply radiofrequency energy to tissue to create tissue lesions at different tissue levels and alter the structure of the tissue without ablating or burning the tissue, while avoiding undertreatment of tissue. The system includes a flexible outer tube, an expandable basket, and a plurality of electrodes with distal tips that can be aligned in a consistent and uniform manner to prevent overheating of tissue and ablation of tissue. International Patent Application Publication No. WO 2007/005830A2 provides an ablation element which comprises an elongated cannula having a proximal end and a distal end defining an internal lumen and a plurality of conductors contained within the cannula. A plurality of ablation stylets are each coupled to the distal end of a respective conductor and are mounted for axial movement. A trocar point is defined proximate the distal end of the cannula and has a deflection surface positioned between the trocar point and the proximal end of the cannula for deflecting, the stylets in a direction from the proximate end of the cannula to the distal end of the cannula to define an ablation volume.

### SUMMARY

An objective of the present invention is to provide a radio-frequency ablation catheter, and a radio-frequency ablation system as defined in independent claim 1. Preferred embodiments are disclosed in the dependent claims.

During the radio-frequency ablation process, the stretching and retraction of a claw electrode can be controlled by pushing a sliding button on a handle to move back and forth. This realizes the auxiliary positioning of an electrode body, the determination of the situation around the electrode body and also by the claw electrode. Technical Solution

An embodiment of the present invention provides a detection mechanism, in a radio-frequency ablation catheter. The detection mechanism comprises a fixing base, a traction wire, a connecting base and multiple claw-shaped electrodes.

The fixing base is slidably mounted in the radio-frequency ablation catheter;
and provided with multiple mounting holes, wherein each of the claw-shaped electrodes extends through the mounting hole and is fixedly connected to the fixing base.

A distal end of the traction wire is fixed on the fixing base, and a proximal end of the traction wire is fixed on a sliding button of the radio-frequency ablation catheter.

The connecting base is fixedly mounted in the radio-frequency ablation catheter; and provided with multiple guide holes, wherein the openings of adjacent guide holes extend in the same direction, each of the claw-shaped electrodes extends through the guide holes, and the guide hole allows the claw-shaped electrode to extend out of the connecting base in a dispersed manner.

Each of the claw-shaped electrodes includes: a first section, a second section, and a third section, wherein the second section is at an obtuse angle with respect to the first section, and the third section is at an obtuse angle with respect to the second section. When the claw-shaped electrode extends out of the connecting base in a dispersed manner, the first section, the second section, and the third section are opened. Distal ends of the claw-shaped electrodes are located at the same latitude.

In a feasible implementation, the detection mechanism also includes a fixing ring, having a distal end fixedly arranged on the claw electrode, and allowing a wire and the claw-shaped electrode to be electrically connected.

In a feasible implementation, the distance between adjacent guide holes of the detection mechanism is the same.

In a feasible implementation, the distance between opposing guide holes of the detection mechanism is different.

The present invention provides a radio-frequency ablation catheter, which includes a handle portion, a needle tube portion, a central electrode, and any detection mechanism as described above.

The needle tube portion includes a first tube sleeve and a second tube sleeve, wherein the connecting base is specifically mounted between the first tube sleeve and the second tube sleeve, the fixing base is specifically slidably mounted in the first tube sleeve, and the fixing ring and the multiple claw-shaped electrodes are located inside the first tube sleeve.

The handle portion comprises a cylinder sleeve and a sliding button, wherein
the sliding button is slidably mounted on the cylinder sleeve; and
a proximal end of the traction wire is fixed on the sliding button.

The central electrode comprises an electrode body, an electrode wire, and an electrode connector, wherein
the electrode body is provided at a distal end of the second tube sleeve, a distal end of the electrode wire is electrically connected to the electrode body, the electrode wire extends through the cylinder sleeve, the first tube sleeve and the second tube sleeve, a proximal end of the electrode wire is electrically connected to the electrode connector, and the electrode connector is located outside the cylinder sleeve.

In a feasible implementation, the radio-frequency ablation catheter also includes: a liquid injection joint and a liquid injection tube, wherein the liquid injection tube extends through the cylinder sleeve, the fixing base and the connecting base, the electrode body is provided with a liquid inlet, and a distal end of the liquid injection tube is in communication with the liquid inlet; and
the liquid injection joint is provided at a proximal end of the liquid injection tube, and located outside the cylinder sleeve.

In a feasible implementation, sprinkler channels are provided in the electrode body of the radio-frequency ablation catheter, wherein the sprinkler channels are in communication with the liquid inlet, and the sprinkler channels are used to dispersely feed the liquid at the liquid inlet.

In a feasible implementation, the electrode body of the radio-frequency ablation catheter is externally provided with an infiltration hood, wherein the infiltration hood includes a plurality of dispersing holes distributed in a rectangular array, and the hole diameters of each row of dispersing holes decrease in a direction from the proximal end to the distal end.

In a feasible implementation, the dispersing hole and the sprinkler channel of the radio-frequency ablation catheter are arranged misaligned.

In a feasible implementation, the electrode body of the radio-frequency ablation catheter includes a cylindrical portion and a tapered portion, wherein the tapered portion is located at a distal end of the cylindrical portion; and the infiltration hood is mounted around the cylindrical portion.

In a feasible implementation, the electrode body of the radio-frequency ablation catheter is provided with a temperature sensor and a signal conduit therein, and
a wiring hole is provided on the electrode body, wherein the temperature sensor is located in the tapered portion, the signal conduit extends through the wiring hole, an insulating layer is provided outside the signal conduit, a distal end of the signal conduit is electrically connected to the temperature sensor, and a proximal end of the signal conduit is electrically connected to the electrode connector.

In a feasible implementation, a slot is provided on the electrode body of the radio-frequency ablation catheter, and the distal end of the second tube sleeve is inserted into the slot.

In a feasible implementation, the infiltration hood of the radio-frequency ablation catheter is made of a high-temperature resistant insulating material.

In a feasible implementation, the electrode body of the radio-frequency ablation catheter is made of fibers.

In a feasible implementation, the second tube sleeve of the radio-frequency ablation catheter is provided with an internal thread, the connecting base is provided with an external thread, and the second tube sleeve and the connecting base are threadedly connected.

In a feasible implementation, the first tube sleeve of the radio-frequency ablation catheter is provided with a counterbore, a threaded hole is provided at the proximal end of the connecting base, and the first tube sleeve and the connecting base are connected by a bolt.

In a feasible implementation, the connecting base includes: a liquid injection hole, wherein the liquid injection hole is positioned along the center line of the connecting base, the liquid injection tube extends through the liquid injection hole, and the guide holes are symmetrically distributed with the liquid injection hole as a center. The guide hole includes a straight section and an arc section, wherein the arc section is located at a distal end of the straight section.

In a feasible implementation, an anti-skid pattern is provided on an inner side wall of the straight section of the guide hole of the radio-frequency ablation catheter.

In a feasible implementation, an anti-wear cushion is provided on an inner side wall of each arc section of the radio-frequency ablation catheter, wherein the anti-wear cushion is made of a rubber material.

In a feasible implementation, the connecting base of the radio-frequency ablation catheter is made of glass fibers.

In a feasible implementation, a mounting post is provided at the proximal end of the connecting base of the radio-frequency ablation catheter, and a spring is mounted around the mounting post, wherein when the fixing base moves toward the connecting base, a distal end of the spring presses against the connecting base, and a proximal end of the spring presses against the fixing base.

In a feasible implementation, the multiple claw-shaped electrodes of the radio-frequency ablation catheter are hollow tubes.

In a feasible implementation, an insulating layer is provided around an outer surface of the first sections of the claw-shaped electrodes of the radio-frequency ablation catheter.

In a feasible implementation, an insulating layer is provided around an outer surface of the second sections of the claw-shaped electrodes of the radio-frequency ablation catheter.

In a feasible implementation, the first section and the second section of the radio-frequency ablation catheter are both straight sections, and the third section is an arc section.

In a feasible implementation, a first groove is respectively provided on both sides of the fixing base of the radio-frequency ablation catheter, a fixture block is provided at the distal end of the traction wire, and the fixture block is inserted in the first groove, to allow the traction wire and the fixing base to be fixedly connected.

In a feasible implementation, the fixing base of the radio-frequency ablation catheter is made of an electric ceramic material.

In a feasible implementation, the fixing ring and the fixing base of the radio-frequency ablation catheter are joined by interference fit to limit the extending length of the claw-shaped electrode.

In a feasible implementation, the fixing base of the radio-frequency ablation catheter is made of a silicone rubber material.

In a feasible implementation, the fixing ring of the radio-frequency ablation catheter is a hollow cylinder, and the fixing ring is provided with a slit.

In a feasible implementation, both ends of the fixing ring of the radio-frequency ablation catheter are provided with a buffering cushion.

In a feasible implementation, the buffering cushion of the radio-frequency ablation catheter is made of an elastic rubber material.

In a feasible implementation, the fixing base of the radio-frequency ablation catheter includes a female fixing base and a male fixing ring joined to each other.

In a feasible implementation, the female fixing ring and the male fixing ring of the radio-frequency ablation catheter are hinged by a hinge shaft.

An embodiment of the present invention also provides a radio-frequency ablation system, which includes the radio-frequency ablation catheter described in any of the foregoing feasible implementations.

### Beneficial effects of the invention

Based on the above implementations, it can be known that the present invention provides a detection mechanism, in a radio-frequency ablation catheter, and a radio-frequency ablation system. The radio-frequency ablation catheter includes a handle portion, a needle tube portion, a central electrode, and a detection mechanism. The needle tube portion includes a first tube sleeve and a second tube sleeve, the handle portion includes a cylinder sleeve and a sliding button, the central electrode includes an electrode body, an electrode wire, and an electrode connector, and the detection mechanism includes a fixing ring, a fixing base, a traction wire, a connecting base, and multiple claw-shaped electrodes. For the detection mechanism, the radio-frequency ablation catheter, and the radio-frequency ablation system of the present invention, with a user as a reference, the end close to the user is the proximal end and the end away from the user is the distal end. The connecting base is mounted between the first tube sleeve 11 and the second tube sleeve, and the fixing base is located at a proximal end of the connecting base. The fixing base, the fixing ring and the multiple claw-shaped electrodes are all provided in the first tube sleeve. The fixing base can slidably move in the first tube sleeve, the fixing ring is mounted at a proximal end of the fixing base and fixed to the claw-shaped electrode, and the claw-shaped electrode extends in the fixing base. The claw-shaped electrodes can slide in the connecting base, and when the claw-shaped electrodes extend out of the connecting base, the claw-shaped electrodes stretch out, and are on the same latitude. The sliding button can slide on the surface of the cylinder sleeve, a proximal end of the traction wire is fixed on the sliding button; and a distal end of the traction wire is fixed to the fixing base. The electrode body is located at a distal end of the second tube sleeve, a distal end of the electrode wire is fixed in the electrode body and electrically connected, a proximal end of the electrode wire is electrically connected to the electrode connector, and the electrode connector is located outside the cylinder sleeve. The multiple claw-shaped electrodes can be pushed in and pushed out of the needle tube portion by the aid of the connecting base, the fixing base, and the traction wire. When the claw-shaped electrode needs to be pushed out of the needle tube portion for detection, the sliding button is pushed distally, so that the sliding button drives the traction wire to move distally, which in turn drives the fixing base to move distally. At this time, the claw-shaped electrodes fixed to the fixing base move distally, as the fixing base moves distally. As a result, the claw-shaped electrodes previously received in the connecting base are pushed out of the needle tube portion. When there is no need to use the claw-shaped electrodes for detection, the sliding button is pushed proximally, so the sliding button drives the traction wire to move proximally, which in turn drives the fixing base to move proximally, so as to pull the claw-shaped electrodes back. At this time, the claw-shaped electrodes previously located outside the needle tube portion are retracted inside the connecting base again. By means of the sliding button, the traction wire and the fixing base 5 are driven to move to finally realize the push-out and retraction of the claw-shaped electrodes 7. This facilitates the control of the claw-shaped electrodes by a user during surgery. During the radio-frequency ablation process, the stretching and retraction of the claw electrodes can be controlled by pushing the sliding button on the handle to move backward and forward. This realizes the auxiliary positioning of an electrode body, the determination of the situation around the electrode body by the claw electrode, and thus the determination of the progress of ablation.

### BRIEF DESCRIPTION OF THE DRAWINGS

To describe the technical solutions according to the embodiments of the present invention or in the prior art more clearly, the drawings needed to be used in the embodiments or in the present art will be described briefly below. Apparently, the drawings in the following description show some embodiments of the present application. Other drawings can be obtained by persons of ordinary skill in the art based on these drawings without creative efforts.
FIG. 1 is a schematic structural view showing the overall structure of a radio-frequency ablation catheter according to a first embodiment of the present invention;
FIG. 2 is a partially enlarged view of a needle tube portion in the radio-frequency ablation catheter according to the first embodiment of the present invention;
FIG. 3 shows a fixing base connected to a fixing ring in the radio-frequency ablation catheter according to the first embodiment of the present invention;
FIG. 4 is a schematic structural view of the fixing base in the radio-frequency ablation catheter according to the first embodiment of the present invention;
FIG. 5 is a schematic view showing the positions of the connecting base and a spring in the radio-frequency ablation catheter according to the first embodiment of the present invention;
FIG. 6 is a first sectional view of the connecting base in the radio-frequency ablation catheter according to the first embodiment of the present invention;
FIG. 7 is a second sectional view of the connecting base in the radio-frequency ablation catheter according to the first embodiment of the present invention;
FIG. 8 is a schematic structural view showing the overall structure of an infiltration hood in the radio-frequency ablation catheter according to the first embodiment of the present invention;
FIG. 9 is a sectional view of the infiltration hood in the radio-frequency ablation catheter according to the first embodiment of the present invention;
FIG. 10 is a schematic view showing a second tube sleeve connected to the infiltration hood in the radio-frequency ablation catheter according to the first embodiment of the present invention;
FIG. 11 is a left side view of the fixing base in the radio-frequency ablation catheter according to the first embodiment of the present invention; and
FIG. 12 is a schematic structural view of a claw-shaped electrode in the radio-frequency ablation catheter according to the first embodiment of the present invention.

### Numeral list:

1. needle tube portion; 11. first tube sleeve; 111. counterbore; 12. second tube sleeve; 121. internal thread; 2. handle portion; 21. cylinder sleeve; 22. sliding button; 23. electrode connector; 24. liquid injection joint; 25. liquid injection tube; 26. electrode wire; 3. central electrode; 31. electrode body; 311. liquid inlet; 312. sprinkler channel; 32. infiltration hood; 321. dispersing holes; 33. temperature sensor; 34. wiring hole; 35. slot; 4. fixing ring; 41. slit; 42. buffering cushion; 5. fixing base; 51. mounting holes; 52. through hole; 53. first groove; 54. fixture block; 55. male fixing ring; 551. bump; 56. female fixing ring; 561. second groove; 57. hinge shaft; 6. connecting base; 61. guide hole; 611. straight section; 6111. anti-slid pattern; 612. arc section; 6121. anti-wear cushion; 62. liquid injection hole; 63. external thread; 64. threaded hole; 65. mounting hole; 7. claw-shaped electrode; 71. first section; 72. second section; 73. third section; 8. spring; 9. insulating layer.

### DESCRIPTION OF THE EMBODIMENTS

To make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application. All other embodiments obtained by persons of ordinary skill in the art based on the embodiments of the present invention without creative efforts shall fall within the protection scope of the present invention.

In the description of the present invention, it needs to be understood that the terms "center", "longitudinal", "transverse", "upper", "lower", "front", "rear", "left", "right", "vertical", "horizontal", "top" , "bottom", "inner", "outer", "axial", "radial", "circumferential" and others indicate directional or position relations based on the directional or position relations shown in the drawings, and are provided to facilitate and simplify the description of the present invention, instead of indicating or implying that the device or element referred to needs to have a specific orientation, and be constructed and operated in a specific orientation. Therefore, these terms cannot be understood as the restrictions to the present invention.

In the present invention, unless otherwise clearly specified and defined, the terms "mounted", "connection", "connected", "fixed" and others should be understood in a broad sense. For example, the connection may be fixed connection or detachable connection, or forms a one-piece structure; may be mechanical connection, electrical connection, or communication connection; may be direct connection, or indirect connection via an intermediate structure; or may be the internal connection of two elements or the interaction relationship between two elements, unless otherwise clearly defined. For those of ordinary skill in the art, specific meanings of the above terms in the present invention can be understood according to specific circumstances. Hereinafter, the technical solutions of the present invention will be described in detail below in connection with specific embodiments. The following specific embodiments can be combined with each other, in which the same or similar concepts or processes may not be repeated in some embodiments.

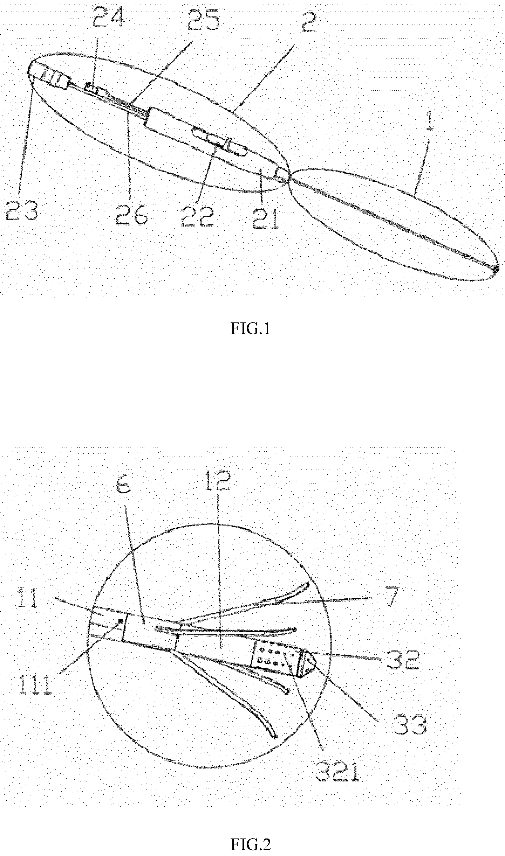

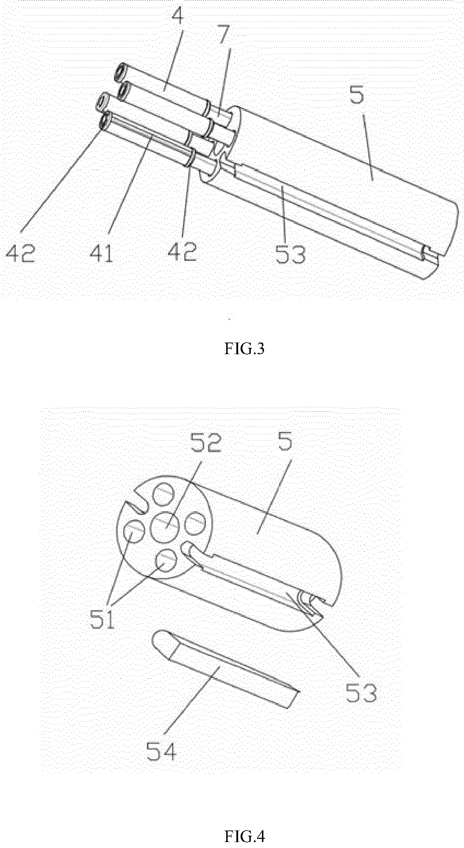

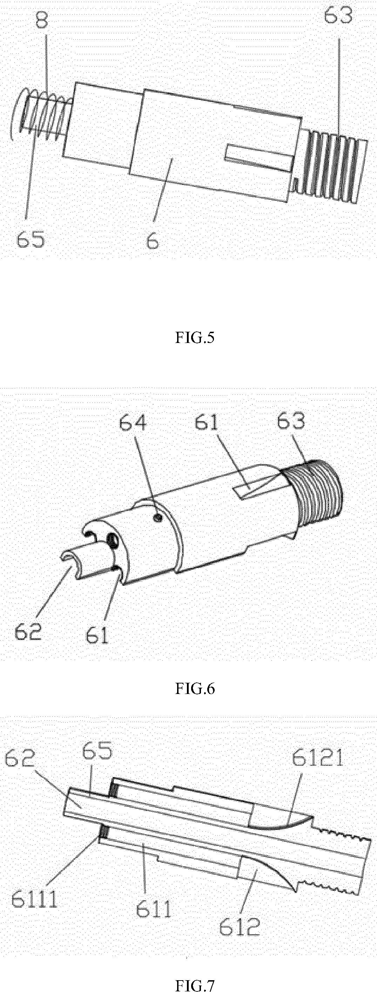

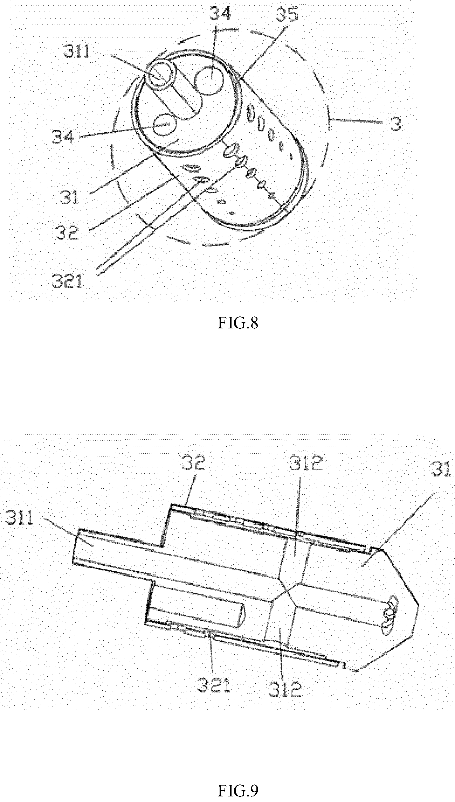

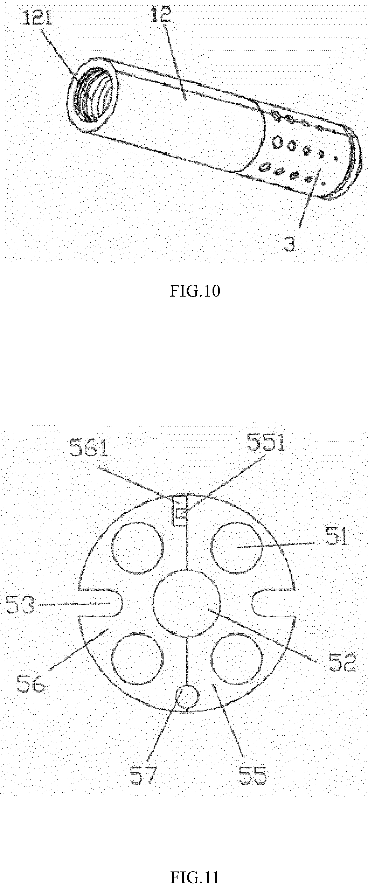

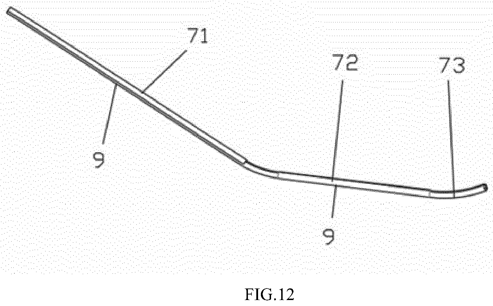

FIG. 1 is a schematic structural view showing the overall structure of a radio-frequency ablation catheter according to a first embodiment of the present invention; FIG. 2 is a partially enlarged view of a needle tube portion in the radio-frequency ablation catheter according to the first embodiment of the present invention; FIG. 3 shows a fixing base connected to a fixing ring in the radio-frequency ablation catheter according to the first embodiment of the present invention; FIG. 4 is a schematic structural view of the fixing base in the radio-frequency ablation catheter according to the first embodiment of the present invention; FIG. 5 is a schematic view showing the positions of the connecting base and a spring in the radio-frequency ablation catheter according to the first embodiment of the present invention; FIG. 6 is a first sectional view of the connecting base in the radio-frequency ablation catheter according to the first embodiment of the present invention; FIG. 7 is a second sectional view of the connecting base in the radio-frequency ablation catheter according to the first embodiment of the present invention; FIG. 8 is a schematic structural view showing the overall structure of an infiltration hood in the radio-frequency ablation catheter according to the first embodiment of the present invention; FIG. 9 is a sectional view of the infiltration hood in the radio-frequency ablation catheter according to the first embodiment of the present invention; FIG. 10 is a schematic view showing a second tube sleeve connected to the infiltration hood in the radio-frequency ablation catheter according to the first embodiment of the present invention; FIG. 11 is a left side view of the fixing base in the radio-frequency ablation catheter according to the first embodiment of the present invention; and FIG. 12 is a schematic structural view of a claw-shaped electrode in the radio-frequency ablation catheter according to the first embodiment of the present invention.

As shown in FIG. 1, a radio-frequency ablation catheter according to an embodiment includes a handle portion 2, a needle tube portion 1, a central electrode 3 and a detection mechanism. The needle tube portion 1 includes a first tube sleeve 11 and a second tube sleeve 12. The handle portion 2 includes a cylinder sleeve 21 and a sliding button 22. The central electrode 3 includes an electrode body 31, an electrode wire 26, and an electrode connector 23. The detection mechanism includes a fixing ring 4, a fixing base 5, a traction wire (not shown), a connecting base 6, and multiple claw-shaped electrodes 7. With a user as a reference, the end close to the user is the proximal end and the end away from the user is the distal end. The radio-frequency ablation catheter includes sequentially, from a proximal end to a distal end, the cylinder sleeve 21, the first tube sleeve 11, the fixing ring 4, the fixing base 5, the connecting ring 6, the second tube sleeve 12, and the central electrode 3. The cylinder sleeve 21 is located at a proximal end of the needle tube portion 1, the first tube sleeve 11 is sleeved at a distal end of the cylinder sleeve 21, the electrode connector 23 is located at a proximal end of the cylinder sleeve 21, a proximal end of the electrode wire 26 is electrically connected to the electrode connector 23, and a distal end of the electrode wire 26 is inserted into the electrode body 31, and electrically connected to the electrode body 31. The electrode body 31 is located at a distal end of the needle tube portion 1, and fixed at a distal end of the second tube sleeve 12. The electrode body 31 is made of fibers. The electrode wire 26 extends through the cylinder sleeve 21, the first tube sleeve 11, the fixing base 5, the connecting base 6, the second tube sleeve 12 and the electrode body 31, respectively.

As shown in FIG. 12, the claw-shaped electrode 7 includes a first section 71, a second section 72, and a third section 73. The first section 71 is a proximal section, the second section 72 is a middle section, and the third section 73 is a distal section. The first section 71 is at an obtuse angle with respect to the second section 72, and the second section 72 is at an obtuse angle with respect to the third section 73. The claw-shaped electrode can obtain an impedance value of a contact position, or the claw-shaped electrode itself is made of a heat-sensitive material, to obtain the highest temperature around the claw-shaped electrode. In other words, by means of the claw-shaped electrode, the detection mechanism can detect the temperature or impedance at the claw-shaped electrode, and thus determine the progress of ablation.

As shown in FIG. 4, the fixing base 5 is located in the first tube sleeve 11, and the fixing base 5 can slidably move in the first tube sleeve 11. Four mounting holes 51 are provided on the fixing base 5, and the four mounting holes 51 are used to fix the claw-shaped electrodes 7. Optionally, the claw-shaped electrode 7 is fixed in the mounting hole 51 by dispensing. That is, the claw-shaped electrode 7 is fixedly provided in the mounting hole 51 by an adhesive. Alternatively the claw-shaped electrode 7 is fixed in the mounting hole 51 by welding. That is, the claw-shaped electrode 7 is welded to the mounting hole 51. A through hole 52 is provided along the center line of the fixing base 5, and the four mounting holes 51 are symmetrically distributed with the through hole 52 as the center. The through hole 52 is provided for accommodating a signal conduit and a liquid injection tube 25. The traction wire (not shown) is fixed at a distal end of the fixing base 5.

As shown in FIG. 3 , the fixing ring 4 is located in the first tube sleeve 11 at a proximal end of the fixing base 5, a distal end of the fixing ring 4 is fixedly on the claw electrode 7, and a proximal end of the fixing ring 4 is used to fix an external wire, and allows the wire and the claw-shaped electrode to be electrically connected. The external wire extends through the cylinder sleeve 21 and the first tube sleeve 11, a proximal end of the external wire is fixed to the electrode connector 23, and a distal end of the external wire is fixed to the fixing ring 4. The first section 71 of the claw-shaped electrode 7 extends out of the fixing base 5, and enter the inside of the fixing ring 4. The distal end of the external wire enters the inside of the fixing ring 4 from a proximal end of the fixing ring 4. In the fixing ring 4, the first section 71 of the claw-shaped electrode 7 is in contact with the distal end of the external wire, to form an electrical connection. The electrode connector 23 is externally connected to a radio-frequency ablation system. A current is flowed to the claw-shaped electrode 7 through the external wire, and then the current is released into the human tissue by the claw-shaped electrode 7. The sliding button 22 is mounted on the surface of the cylinder sleeve 21, and the sliding button 22 can slidably move on the surface of the cylinder sleeve 21. A distal end of the traction wire (not shown) is fixed to the fixing base 5, and a proximal end of the traction wire (not shown) is fixed to the sliding button 22. By pushing the sliding button 22 to move forward or backward, the traction wire is driven to move forward or backward. Since one end of the traction wire is fixed to the fixing base 5, the traction wire (not shown) drives the fixing base 5 to slide in the first tube sleeve 11. The claw-shaped electrode 7 is fixed in the fixing base 5. As the fixing base 5 slides forward or backward, the claw-shaped electrode 7 fixed in the fixing base 5 will also move forward or backward with the fixing base 5. Optionally, the claw-shaped electrode 7 is fixedly connected to the fixing base 5 by an adhesive, and they move as a whole.

As shown in FIG. 7 , the connecting base 6 is located between the first tube sleeve 11 and the second tube sleeve 12, and the first tube sleeve 11 and the second tube sleeve 12 are respectively fixed at a proximal and distal end of the connecting base 6. Four guide holes 61 are provided on the connecting base 6, and the openings of each two adjacent guide holes 61 extend in same direction. The four guide holes 61 are respectively located in four directions on the connecting base 6, and the four guide holes 61 are distributed in a circumferential array with the central axis of the connecting base 6 as a center. Therefore, among the four guide holes 61, the distance between each two adjacent guide holes 61 is the same; and the distance between each pair of opposing guide holes 61 is different. The claw-shaped electrodes 7 slide through the guide holes 61 in the connecting base 6, and these guide holes 61 make the claw-shaped electrodes 7 extend out of the connecting base 6 in a dispersed manner. The four claw-shaped electrodes 7 are separate from each other and do not interfere with each other. Since the claw-shaped electrode 7 is divided into the first section 71, the second section 72 and the third section 73, when the claw-shaped electrode 7 extends out of the connecting base 6, the first section 71 is still located inside the guide hole 61, and the second section 72 and the third section 73 extend out of the guide hole 61 of the connecting base 6 under the pushing force of the fixing base 5, Because of the obtuse angles between the first section 71 and the second section 72 and between the second section 72 and the third section 73, the claw-shaped electrodes 7 stretch out when they extend out of the connecting base 6, and they have the same length when they extend out of the needle tube portion 1. Since the positions of the four guide holes 61 in the connecting base 6 correspond to each other and the positions of the distal openings of the guide holes 61 are the same relative to the connecting base 6, when the claw-shaped electrodes 7 extend out of the guide holes 61, the distal ends of the claw-shaped electrodes 7 are at the same latitude.

The radio-frequency ablation catheter is used in a radio-frequency ablation system. The radio-frequency ablation system includes a radio-frequency generator (ablation instrument), wherein the radio-frequency generator (ablation instrument) is used to connect to the radio-frequency ablation catheter, and provides an electrical signal to the electrode connector of the radio-frequency ablation catheter, to allow the central electrode and the claw-shaped electrode to work.

In view of the foregoing description, it can be easily found that the radio-frequency ablation catheter of the present invention includes a handle portion 2, a needle tube portion 1, a central electrode 3, and a detection mechanism. The needle tube portion 1 includes a first tube sleeve 11 and a second tube sleeve 12. The handle portion 2 includes a cylinder sleeve 21 and a sliding button 22. The central electrode 3 includes an electrode body 31, an electrode wire 26, and an electrode connector 23. The detection mechanism includes a fixing ring 4, a fixing base 5, a traction wire, a connecting base 6, and multiple claw-shaped electrodes 7. For the detection mechanism, radio-frequency ablation catheter, and radio-frequency ablation system of the present invention, with a user as a reference, the end close to the user is the proximal end and the end away from the user is the distal end. The connecting base 6 is mounted between the first tube sleeve 11 and the second tube sleeve 12, and the fixing base 5 is located at a proximal end of the connecting base 6. The fixing base 5, the fixing ring 4 and the multiple claw-shaped electrodes 7 are all provided in the first tube sleeve 11. The fixing base 5 can slidably move in the first tube sleeve 11, the fixing ring 4 is mounted at a proximal end of the fixing base 5 and fixed to the claw-shaped electrode 7, and the claw-shaped electrodes 7 are inserted in the fixing base 5. The claw-shaped electrodes 7 can slide in the connecting base 6, and when the claw-shaped electrodes 7 extend out of the connecting base 6, the claw-shaped electrodes 7 stretch out, and are on the same latitude. The sliding button 22 can slide on the surface of the cylinder sleeve 21. A proximal end of the traction wire is fixed to the sliding button 22, and a distal end of the traction wire is fixed to the fixing base 5. The electrode body 31 is located at a distal end of the second tube sleeve 12, a distal end of the electrode wire 26 is fixed in the electrode body 31 and electrically connected, a proximal end of the electrode wire 26 is electrically connected to the electrode connector 23, and the electrode connector 23 is located outside the cylinder sleeve 21. The multiple claw-shaped electrodes 7 can be pushed in and pushed out of the needle tube portion 1 by the aid of the connecting 6, the fixing base 5, and the traction wire. When the claw-shaped electrode 7 needs to be pushed out of the needle tube portion 1 for detection, the sliding button 22 is pushed distally, so the sliding button 22 drives the traction wire to move distally, which in turn drives the fixing base 5 to move distally. At this time, the claw-shaped electrodes 7 fixed to the fixing base 5 move distally, as the fixing base 5 moves distally. As a result, the claw-shaped electrodes 7 previously received in the connecting base 6 are pushed out of the needle tube portion 1. When there is no need to use the claw-shaped electrodes 7 for detection, the sliding button 22 is pushed proximally, so the sliding button 22 drives the traction wire to move proximally, which in turn drives the fixing base 5 to move proximally, so as to pull the claw-shaped electrodes 7 back. At this time, the claw-shaped electrodes 7 previously located outside the needle tube portion 1 are retracted inside the connecting base 6 again. By means of the sliding button 22, the traction wire and the fixing base 5 are driven to move, to finally realize the push-out and retraction of the claw-shaped electrodes 7. This facilitates the control of the claw-shaped electrodes 7 by a user during surgery. When this detection device is used during surgery, the needle tube portion is inserted into the human body, for example, into the human trachea, then the claw-shaped electrodes are stretched out by using the sliding button, and the claw-shaped electrodes prop against the inner wall of human trachea, to stabilize the central electrode. At the same time, the claw-shaped electrodes can obtain the temperature or impedance data of the human trachea, so as to get the progress of ablation at the central electrode. Therefore, the detection device not only realizes the auxiliary positioning of the electrode body, but also get the progress of ablation by determining the temperature or impedance of the claw-shaped electrodes by the claw-shaped electrodes.

Optionally, in this embodiment, the radio-frequency ablation catheter also includes: a liquid injection joint 24 and a liquid injection tube 25, wherein the liquid injection joint 24 is located at a proximal end of the cylinder sleeve 21, and the liquid injection j oint 24 is connected to an external injector. A proximal end of the liquid injection tube 25 is connected to the liquid injection joint 24, and a distal end of the liquid injection tube 25 is inserted into the electrode body 31. The liquid injection tube 25 is inserted in the cylinder sleeve 21, the first tube sleeve 11 and the second tube sleeve 12, and extends through the cylinder sleeve 21, the fixing base 5 and the connecting base 6 respectively. A liquid inlet 311 is provided on the electrode body 31, wherein the liquid inlet 311 is located in the middle of the electrode body 31, and the liquid inlet 311 is connected to a distal end of the liquid injection tube 25. Saline is discharged from the injector, flows through the liquid injection joint 24, the liquid injection tube 25 and the liquid inlet 311, and finally enters the interior of the electrode body 31.

As shown in FIG. 9 , optionally, in this embodiment, the electrode body 31 includes: a cylindrical portion and a tapered portion, wherein the tapered portion is located at a distal end of the cylindrical portion. An infiltration hood 32 is provided outside the electrode body 31, wherein the infiltration hood 32 is made of high-temperature resistant insulating material, and the infiltration hood 32 is mounted around the cylindrical portion of the electrode body 31. Sprinkler channels 312 are provided inside the electrode body 31, and two sets of sprinkler channels 312 are distributed in a cross shape. The two sets of sprinkler channels are in communication with the liquid inlet 311. The saline flowing through the liquid injection tube 25 is concentrated in the liquid inlet 311 and then dispensed into the sprinkler channels 312. On the surface of the infiltration hood 32, multiple dispersing holes 321 are provided in a rectangular pattern, and the hole diameters of each row of dispersing holes 321 decrease in a direction from the proximal end to the distal end. The multiple dispersing holes 321 are in communication with the sprinkler channels 312, and arranged misaligned with the sprinkler channels 312. There is a gap between the infiltration hood 32 and the surface of the electrode body 31, and the saline in the sprinkler channel 312 flows through the gap to the infiltration hood 32. Due to the multiple dispersing holes 321 present on the surface of the infiltration hood 32, the saline flows out through the dispersing holes 321, to diffuse inside the body tissue.

Optionally, in this embodiment, the electrode body 31 is also provided with a temperature sensor 33 and a signal conduit therein. A wiring hole 34 is provided on the electrode body 31, and the wiring hole 34 is located on the side with the liquid inlet 311, wherein the signal conduit is inserted into the wiring hole 34. The temperature sensor 33 is located on the surface of the tapered portion of the electrode body 31, the temperature sensor 33 is electrically connected to a distal end of the signal conduit, and a proximal end of the signal conduit is fixed on the electrode connector 23 and electrically connected to the electrode connector 23. A rubber insulating layer 9 is provided outside the signal conduit. The temperature sensor 33 can be a thermistor, which is sensitive to temperature, and shows different resistance at different temperatures, wherein the resistance decreases as the temperature increases. After the saline is infiltrated into the human tissue from the dispersing holes 321, the ablation process starts. With the continuous increase of ablation saline, the ablation area continues to increase, and the temperature of the ablation area changes constantly. When the temperature becomes higher and higher, the resistance of the thermistor in a local area decreases as the temperature increases, and the thermistor and the signal conduit are electrically conducted. When the resistance of the thermistor changes, the resistance change is transmitted to the radio-frequency ablation system by the signal conduit (not shown). A distal end of the signal conduit is connected to the thermistor, and a proximal end of the signal conduit is fixed on the electrode connector 23. According to the resistance change on the radio-frequency ablation system, the local temperature change corresponding to the resistance is calculated. In this way, the temperature can be controlled by the flow rate of the brine. Moreover, the temperature change inside human tissue can be intuitively observed on the radio-frequency ablation system.

As shown in FIG. 8, optionally, in this embodiment, a slot 35 is provided at a proximal end of the electrode body 31, and an outer wall of the second tube sleeve 12 is inserted into the slot 35, to fix the electrode body 31 and the second tube sleeve 12 together. An internal thread 121 is provided at a proximal end of the second tube sleeve 12, an external thread 63 is provided at the distal end of the connecting base 6, and the connecting base 6 and the second tube sleeve 12 are threadedly connected.

Optionally, in this embodiment, a threaded hole 64 is provided at the proximal end of the connecting base 6, a counterbore 111 is provided at a distal end of the first tube sleeve 11, and a bolt is inserted into the counterbore 111 and the threaded hole 64 to fix the connecting base 6 and the first tube sleeve 11 together. As the bolt is continuously tightened, the connecting base 6 and the first tube sleeve 11 are fastened. When the bolt is fully screwed into the threaded hole 64, the surface of the bolt is parallel to the surface of the connecting base 6, and the bolt is tucked into the counterbore 111.

Optionally, in this embodiment, a liquid injection hole 62 is provided along the center line of the connecting base 6. The liquid injection tube 25 and the signal conduit are inserted in the liquid injection hole 62. The four guide holes 61 on the connecting base 6 are symmetrically distributed with the liquid injection hole 62 as a center. The liquid injection hole 62 is used to define the position of the liquid injection tube 25, making the sliding path of the liquid injection tube 25 not deviate. The guide hole 61 includes a straight section 611 and an arc section 612, wherein the straight section 611 is located at a proximal end of the arc section 612. Since the first section 71, the second section 72 and the third section 73 of the claw-shaped electrode 7 are connected and have an obtuse angle therebetween, the connection between the straight section 611 and the arc section 612 of the guide hole 61 can facilitate the claw-shaped electrode 7 to slide. The arc-shaped section 612 is convenient for the independent and smooth stretching and retraction of the claw-shaped electrode 7, to increase the supporting force and stretching range of the claw-shaped electrode 7. An anti-skid pattern 6111 is provided on an inner side wall of the straight section 611 of the guide hole 61. When the claw-shaped electrode 7 extends out of the needle tube portion 1, the anti-skid pattern 6111 in the straight section 611 acts to fix the position of the claw-shaped electrode 7 to keep it stable. An anti-wear cushion 6121 made of a silicone material is provided on the inner side wall of each arc section 612, and the anti-wear cushion 6121 is located between the arc-shaped section 612 and the claw-shaped electrode 7. When the claw-shaped electrode 7 is pushed out of or pulled into the connecting base 6, no friction that damages the surface of the claw-shaped electrode 7 occurs between the claw-shaped electrode 7 and the surface of the arc section 612. Therefore, the presence of the anti-wear cushion 6121 can relieve the wear on the surface of the claw-shaped electrode 7.

As shown in FIG. 5 , optionally, in this embodiment, a mounting post 65 is provided at the proximal end of the connecting base 6, and a spring 8 is mounted around the mounting post 65. When the claw-shaped electrode 7 extends out of the needle tube portion 1, the fixing base 5 moves toward the connecting base 6. When the fixing base 5 moves toward the connecting base 6, a distal end of the spring 8 presses against the proximal end of the connecting base 6, and a proximal end of the spring 8 presses against the fixing base 5. The presence of the spring 8 increases the buffer capacity when the claw-shaped electrode 7 extends out of the connecting base 6, and keep the claw-shaped electrode 7 stable. As shown in FIG. 12 , optionally, in this embodiment, the claw-shaped electrode 7 is hollow, and the surfaces of the first section 71 and the second section 72 of the claw-shaped electrode 7 are both provided with an insulating layer 9 made of a rubber material. When a current flows to the claw-shaped electrode 7 via a conductive tube, signal interference is caused. The presence of a layer of rubber on the surface of the claw-shaped electrode 7 can reduce signal interference, by signal shielding. The first section 71 and the second section 72 of the claw-shaped electrode 7 are both a straight section 611, and the third section 73 is an arc section.

As shown in FIG. 4 , optionally, in this embodiment, two first grooves 53 are provided on the surface of the fixing base 5, a fixture block 54 is provided at the distal end of the traction wire, and the first groove 53 is used to fix the traction wire. When the traction wire needs to be clamped into the first groove 53, the fixture block 54 on the traction wire can be directly pushed into the first groove 53, to allow the fixture block 54 on the traction line to be right clamped into the first groove 53. Through the cooperation of the block 54 and the first groove 53, the traction wire is fixed on the fixing base 5.

As shown in FIG. 11 , optionally, in this embodiment, the fixing base 5 includes a male fixing ring 554 and a female fixing ring 564. The fixing base 5 is divided into a front fixing ring 4 and a rear fixing ring 4. The male fixing ring 554 and the female fixing ring 564 are joined to form a complete fixing base 5. The male fixing ring 554 and the female fixing ring 564 are hinged at one end by a hinge shaft 57. A second groove 561 is provided on the other end of the female fixing ring 564, a bump 551 is provided at the other end of the male fixing ring 554, and the male fixing ring 554 and the female fixing ring 564 are fastened by inserting the bump 551 into the second grove 561.

The second embodiment is an alternative to the first embodiment, with the difference that the connecting base 6 is made of a fiberglass material, having good insulation performance, strong heat resistance, high anti-corrosion effect, and high mechanical strength.

The third embodiment is an alternative to the first embodiment, with the difference that the fixing base 5 is made of an electric ceramic material, which is a porcelain insulating material having good insulation performance and mechanical strength, excellent mechanical performance, good electrical performance, and high environmental resistance.

The fourth embodiment is an alternative to the first embodiment, with the difference that the fixing ring 4 is made of a silicone rubber material, having high-temperature stability, and able to maintain a certain degree of flexibility and elasticity in a high-temperature environment.

In the present invention, unless otherwise clearly specified and defined, when a first feature is described to be "on" or "under" a second feature, the first feature and the second feature may be in direct contact, or the first feature and the second feature may be in indirect contact by providing an intermediate structure therebetween.

Further, when a first feature is described to be "above" or "over" a second feature, the first feature may be right above or obliquely above the first feature, or it merely means that the level of the first feature is higher than that of the second feature. When a first feature is described to be "under" or "below" a second feature, the first feature may be right under or obliquely under the first feature, or it merely means that the level of the first feature is lower than that of the second feature.

In the description of the specification, the description with reference to the terms "one embodiment", "some embodiments", "examples", "specific examples" or "some examples", etc., means that specific features, structures, materials or characteristics described in connection with the embodiment or example are embraced in at least one embodiment or example of the present invention. In the specification, the illustrative expression of the above terms is not necessarily referring to the same embodiment or example. Moreover, the described specific features, structures, materials or characteristics may be combined in any suitable manners in one or more embodiments or examples. Furthermore, where no contradiction exists, the various embodiments or examples and features of various embodiments or examples described in this specification can be combined by those skilled in the art.

Finally, it should be noted that the above-described embodiments are merely illustrative of, and not intended to limit the technical solutions of the present invention. Although the present invention has been described in detail with reference to the foregoing embodiments, It should be understood by those of ordinary skill in the art that modifications can be made to technical solutions described in the foregoing embodiments, or equivalent replacements can be made to some or all of the technical features therein, without causing the essence of the corresponding technical solutions to deviate from the scope of the technical solution described in the embodiments of the present invention.

## Claims

1. A radio-frequency ablation catheter, comprising a handle portion (2), a needle tube portion (1), a central electrode (3) for ablation treatment, and a detection mechanism, wherein the detection mechanism comprises:
a fixing base (5), a traction wire, a connecting base (6) and multiple claw-shaped electrodes (7), wherein
the fixing base (5) is provided with multiple mounting holes (51), and each of the claw-shaped electrodes (7) extends through the mounting hole (51) and is fixedly connected to the fixing base (5) respectively;
a distal end of the traction wire is fixed to the fixing base (5);
the connecting base (6) is provided with multiple guide holes (61), wherein the openings of adjacent guide holes (61) extend in the same direction, each of the claw-shaped electrodes (7) extends through the guide hole (61) respectively, and the guide holes (61) allow the claw-shaped electrodes (7) to extend out of the connecting base (6) in a dispersed manner; wherein
each of the claw-shaped electrodes (7) comprises: a proximal first section (71), a middle second section (72), and a distal third section (73), wherein the second section (72) is at an obtuse angle with respect to the first section (71) and bent radially outwardly relative to the first section (71), and the third section (73) is at an obtuse angle with respect to the second section (72) and bent radially outwardly relative to the second section (72), the needle tube portion (1) comprises a first tube sleeve (11) and a second tube sleeve (12), wherein the central electrode (3), the second tube sleeve (12), the connecting base (6) and the first tube sleeve (11) are connected sequentially in a direction from a distal end to a proximal end of the radio-frequency ablation catheter, a proximal end of the connecting base (6) is fixed at a distal end of the first tube sleeve (11) and a distal end of the connecting base (6) is fixed at a proximal end of the second tube sleeve (12), the central electrode (3) is fixed at a distal end of the second tube sleeve (12), the fixing base (5) is slidably mounted in the first tube sleeve (11), and the multiple claw-shaped electrodes (7) are located inside the first tube sleeve (11); wherein when the claw-shaped electrodes (7) extend out of the connecting base (6) in a dispersed manner, the claw-shaped electrodes (7) stretch out and surround the central electrode (3) to obtain the temperature or impedance data around the central electrode (3) so as to monitor the progress of ablation of the central electrode (3), and distal ends of the claw-shaped electrodes (7) are located on the same latitude, and wherein
the handle portion (2) comprises a cylinder sleeve (21) and a sliding button (22),
wherein
the sliding button (22) is slidably mounted on the cylinder sleeve (21), and
a proximal end of the traction wire is fixed on the sliding button (22); and
the central electrode (3) comprises an electrode body (31), an electrode wire, and an electrode connector, wherein
the electrode body (31) is provided at a distal end of the second tube sleeve (12), a distal end of the electrode wire is electrically connected to the electrode body (31), the electrode wire extends through the cylinder sleeve (21), the first tube sleeve (11) and the second tube sleeve (12), a proximal end of the electrode wire is electrically connected to the electrode connector, and the electrode connector is located outside the cylinder sleeve (21).

2. The radio-frequency ablation catheter according to claim 1, further comprising a liquid injection joint (24) and a liquid injection tube (25), wherein
the liquid injection tube (25) extends through the cylinder sleeve (21), the fixing base (5) and the connecting base (6), the electrode body (31) is provided with a liquid inlet (311), and a distal end of the liquid injection tube (25) is in communication with the liquid inlet (311); and
the liquid injection joint (24) is provided at a proximal end of the liquid injection tube (15), and located outside the cylinder sleeve (21).

3. The radio-frequency ablation catheter according to claim 2, wherein sprinkler channels (312) are provided in the electrode body (31), wherein the sprinkler channels (312) are in communication with the liquid inlet (311), and the sprinkler channels (312) are used to dispersely feed the liquid at the liquid inlet (311).

4. The radio-frequency ablation catheter according to claim 3, wherein an infiltration hood (32) is provided outside the electrode body (31), wherein the infiltration hood (32) comprises a plurality of dispersing holes (321) distributed in a rectangular array, and the hole diameters of each row of dispersing holes (321) decrease in a direction from the proximal end to the distal end.

5. The radio-frequency ablation catheter according to claim 1, wherein the connecting base (6) comprises a liquid injection hole (62), wherein the liquid injection hole (62) is positioned along the center line of the connecting base (6), the liquid injection tube (25) extends through the liquid injection hole (62), and the guide holes (61) are symmetrically distributed with the liquid injection hole (62) as a center; and the guide hole 61 comprises a straight section 611() and an arc section (612), wherein the arc section (612) is located at a distal end of the straight section (611).

6. The radio-frequency ablation catheter according to claim 5, wherein the connecting base (6) is made of glass fibers.

7. The radio-frequency ablation catheter according to claim 1, wherein the multiple claw-shaped electrodes (7) are hollow tubes.

8. The radio-frequency ablation catheter according to claim 1, wherein the detection mechanism further comprising a fixing ring (4), wherein a distal end of the fixing ring (4) is fixed on the claw shaped electrode (7), and a proximal end of the fixing ring (4) is externally connected to a wire, allowing the wire and the claw-shaped electrodes (7) to be electrically connected.

9. The radio-frequency ablation catheter according to claim 8, wherein the fixing ring (4) and the fixing base (5) are joined by interference fit to limit the extending length of the claw-shaped electrode (7).

10. The radio-frequency ablation catheter according to claim 1, wherein the second tube sleeve (12) is provided with an internal thread (121), the connecting base (6) is provided with an external thread (63), and the second tube sleeve (12) and the connecting base (6) are threadedly connected.

11. The radio-frequency ablation catheter according to claim 1, wherein the first tube sleeve (11) is provided with a counterbore (111), a threaded hole (64) is provided at the proximal end of the connecting base (6), and the first tube sleeve (11) and the connecting base (6) are connected by a bolt.

12. The radio-frequency ablation catheter according to claim 1, wherein a mounting post is provided at the proximal end of the connecting base (6), and a spring (8) is mounted around the mounting post, wherein when the fixing base (5) moves toward the connecting base (6), a distal end of the spring (8) presses against the connecting base (6), and a proximal end of the spring (8) presses against the fixing base (5).

13. The radio-frequency ablation catheter according to claim 1, wherein the first section (71) and the second section (72) are both straight sections, and the third section (73) is an arc section.

14. The radio-frequency ablation catheter according to claim 1, wherein a first groove (53) is respectively provided on both sides of the fixing base (5), a fixture block (54) is provided at the distal end of the traction wire, and the fixture block (54) is inserted in the first groove (53), to allow the traction wire and the fixing base (5) to be fixedly connected.

15. A radio frequency ablation system, comprising a generator and a radio-frequency ablation catheter according to any one of claims 1 to 14.

## Patentansprüche

1. Hochfrequenzablationskatheter, umfassend einen Griffabschnitt (2), einen Nadelröhrenabschnitt (1), eine Mittelelektrode (3) zur Ablationsbehandlung und einen Erfassungsmechanismus, wobei der Erfassungsmechanismus umfasst:
eine Befestigungsbasis (5), einen Zugdraht, eine Verbindungsbasis (6) und mehrere klauenförmige Elektroden (7), wobei
die Befestigungsbasis (5) mit mehreren Montagelöchern (51) versehen ist und sich jede der klauenförmigen Elektroden (7) durch das Montageloch (51) erstreckt und jeweils fest mit der Befestigungsbasis (5) verbunden ist;
ein distales Ende des Zugdrahts an der Befestigungsbasis (5) befestigt ist;
die Verbindungsbasis (6) mit mehreren Führungslöchern (61) versehen ist, wobei sich die Öffnungen benachbarter Führungslöcher (61) in die gleiche Richtung erstrecken, sich jede der klauenförmigen Elektroden (7) jeweils durch das Führungsloch (61) erstreckt und die Führungslöcher (61) es den klauenförmigen Elektroden (7) ermöglichen, sich in einer verteilten Weise aus der Verbindungsbasis (6) heraus zu erstrecken; wobei:
jede der klauenförmigen Elektroden (7) umfasst: einen proximalen ersten Abschnitt (71), einen mittleren zweiten Abschnitt (72) und einen distalen dritten Abschnitt (73), wobei sich der zweite Abschnitt (72) in einem stumpfen Winkel in Bezug auf den ersten Abschnitt (71) befindet und in Bezug auf den ersten Abschnitt (71) radial nach außen gebogen ist und sich der dritte Abschnitt (73) in einem stumpfen Winkel in Bezug auf den zweiten Abschnitt (72) befindet und in Bezug auf den zweiten Abschnitt (72) radial nach außen gebogen ist, wobei der Nadelröhrenabschnitt (1) eine erste Röhrenhülse (11) und eine zweite Röhrenhülse (12) umfasst, wobei die Mittelelektrode (3), die zweite Röhrenhülse (12), die Verbindungsbasis (6) und die erste Röhrenhülse (11) nacheinander in einer Richtung von einem distalen Ende zu einem proximalen Ende des Hochfrequenzablationskatheters verbunden sind, ein proximales Ende der Verbindungsbasis (6) an einem distalen Ende der ersten Röhrenhülse (11) befestigt ist und ein distales Ende der Verbindungsbasis (6) an einem proximalen Ende der zweiten Röhrenhülse (12) befestigt ist, die Mittelelektrode (3) an einem distalen Ende der zweiten Röhrenhülse (12) befestigt ist, die Befestigungsbasis (5) verschiebbar in der ersten Röhrenhülse (11) montiert ist und sich die mehreren klauenförmigen Elektroden (7) innerhalb der ersten Röhrenhülse (11) befinden; wobei, wenn sich die klauenförmigen Elektroden (7) in einer verteilten Weise aus der Verbindungsbasis (6) heraus erstrecken, sich die klauenförmigen Elektroden (7) ausdehnen und die Mittelelektrode (3) umgeben, um die Temperatur- oder Impedanzdaten um die Mittelelektrode (3) herum zu erhalten, um den Fortschritt der Ablation der Mittelelektrode (3) zu überwachen, und sich distale Enden der klauenförmigen Elektroden (7) auf dem gleichen Breitengrad befinden, und wobei
der Griffabschnitt (2) eine Zylinderhülse (21) und einen Schiebeknopf (22) umfasst,
wobei
der Schiebeknopf (22) verschiebbar an der Zylinderhülse (21) montiert ist und
ein proximales Ende des Zugdrahts an dem Schiebeknopf (22) befestigt ist; und
die Mittelelektrode (3) einen Elektrodenkörper (31), einen Elektrodendraht und einen Elektrodenverbinder umfasst, wobei
der Elektrodenkörper (31) an einem distalen Ende der zweiten Röhrenhülse (12) vorgesehen ist, ein distales Ende des Elektrodendrahts elektrisch mit dem Elektrodenkörper (31) verbunden ist, sich der Elektrodendraht durch die Zylinderhülse (21), die erste Röhrenhülse (11) und die zweite Röhrenhülse (12) erstreckt, ein proximales Ende des Elektrodendrahts elektrisch mit dem Elektrodenverbinder verbunden ist und sich der Elektrodenverbinder außerhalb der Zylinderhülse (21) befindet.

2. Hochfrequenzablationskatheter nach Anspruch 1, ferner umfassend eine Flüssigkeitsinjektionsverbindung (24) und eine Flüssigkeitsinjektionsröhre (25), wobei sich die Flüssigkeitsinjektionsröhre (25) durch die Zylinderhülse (21), die Befestigungsbasis (5) und die Verbindungsbasis (6) erstreckt, der Elektrodenkörper (31) mit einem Flüssigkeitseinlass (311) versehen ist und ein distales Ende der Flüssigkeitsinjektionsröhre (25) mit dem Flüssigkeitseinlass (311) in Verbindung steht; und
die Flüssigkeitsinjektionsverbindung (24) an einem proximalen Ende der Flüssigkeitsinjektionsröhre (15) vorgesehen ist und sich außerhalb der Zylinderhülse (21) befindet.

3. Hochfrequenzablationskatheter nach Anspruch 2, wobei Sprinklerkanäle (312) in dem Elektrodenkörper (31) vorgesehen sind, wobei die Sprinklerkanäle (312) mit dem Flüssigkeitseinlass (311) in Verbindung stehen und die Sprinklerkanäle (312) verwendet werden, um die Flüssigkeit an dem Flüssigkeitseinlass (311) verteilt zuzuführen.

4. Hochfrequenzablationskatheter nach Anspruch 3, wobei eine Infiltrationshaube (32) außerhalb des Elektrodenkörpers (31) vorgesehen ist, wobei die Infiltrationshaube (32) mehrere Verteilungslöcher (321) umfasst, die in einer rechteckigen Anordnung verteilt sind, und die Lochdurchmesser jeder Reihe von Verteilungslöchern (321) in einer Richtung von dem proximalen Ende zu dem distalen Ende abnehmen.

5. Hochfrequenzablationskatheter nach Anspruch 1, wobei die Verbindungsbasis (6) ein Flüssigkeitsinjektionsloch (62) umfasst, wobei das Flüssigkeitsinjektionsloch (62) entlang der Mittellinie der Verbindungsbasis (6) positioniert ist, sich die Flüssigkeitsinjektionsröhre (25) durch das Flüssigkeitsinjektionsloch (62) erstreckt und die Führungslöcher (61) symmetrisch mit dem Flüssigkeitsinjektionsloch (62) als ein Zentrum verteilt sind; und das Führungsloch 61 einen geraden Abschnitt (611) und einen Bogenabschnitt (612) umfasst, wobei sich der Bogenabschnitt (612) an einem distalen Ende des geraden Abschnitts (611) befindet.

6. Hochfrequenzablationskatheter nach Anspruch 5, wobei die Verbindungsbasis (6) aus Glasfasern hergestellt ist.

7. Hochfrequenzablationskatheter nach Anspruch 1, wobei die mehreren klauenförmigen Elektroden (7) Hohlröhren sind.

8. Hochfrequenzablationskatheter nach Anspruch 1, wobei der Erfassungsmechanismus ferner einen Befestigungsring (4) umfasst, wobei ein distales Ende des Befestigungsrings (4) an der klauenförmigen Elektrode (7) befestigt ist und ein proximales Ende des Befestigungsrings (4) extern mit einem Draht verbunden ist, so dass der Draht und die klauenförmigen Elektroden (7) elektrisch verbunden werden können.

9. Hochfrequenzablationskatheter nach Anspruch 8, wobei der Befestigungsring (4) und die Befestigungsbasis (5) durch Presspassung verbunden sind, um die Erstreckungslänge der klauenförmigen Elektrode (7) zu begrenzen.

10. Hochfrequenzablationskatheter nach Anspruch 1, wobei die zweite Röhrenhülse (12) mit einem Innengewinde (121) versehen ist, die Verbindungsbasis (6) mit einem Außengewinde (63) versehen ist und die zweite Röhrenhülse (12) und die Verbindungsbasis (6) durch Gewinde verbunden sind.

11. Hochfrequenzablationskatheter nach Anspruch 1, wobei die erste Röhrenhülse (11) mit einer Senkung (111) versehen ist, ein Gewindeloch (64) an dem proximalen Ende der Verbindungsbasis (6) vorgesehen ist und die erste Röhrenhülse (11) und die Verbindungsbasis (6) durch einen Bolzen verbunden sind.

12. Hochfrequenzablationskatheter nach Anspruch 1, wobei ein Montagepfosten an dem proximalen Ende der Verbindungsbasis (6) vorgesehen ist und eine Feder (8) um den Montagepfosten montiert ist, wobei, wenn sich die Befestigungsbasis (5) in Richtung der Verbindungsbasis (6) bewegt, ein distales Ende der Feder (8) gegen die Verbindungsbasis (6) drückt und ein proximales Ende der Feder (8) gegen die Befestigungsbasis (5) drückt.

13. Hochfrequenzablationskatheter nach Anspruch 1, wobei der erste Abschnitt (71) und der zweite Abschnitt (72) beide gerade Abschnitte sind und der dritte Abschnitt (73) ein Bogenabschnitt ist.

14. Hochfrequenzablationskatheter nach Anspruch 1, wobei eine erste Nut (53) jeweils auf beiden Seiten der Befestigungsbasis (5) vorgesehen ist, ein Befestigungsblock (54) an dem distalen Ende des Zugdrahts vorgesehen ist und der Befestigungsblock (54) in die erste Nut (53) eingesetzt ist, so dass der Zugdraht und die Befestigungsbasis (5) fest verbunden werden können.

15. Hochfrequenzablationssystem, umfassend einen Generator und einen Hochfrequenzablationskatheter nach einem der Ansprüche 1 bis 14.

## Revendications

1. Cathéter d'ablation par radiofréquence, comprenant une partie de poignée (2), une partie de tube d'aiguille (1), une électrode centrale (3) pour un traitement d'ablation, et un mécanisme de détection, dans lequel le mécanisme de détection comprend :
une base de fixation (5), un fil de traction, une base de connexion (6) et de multiples électrodes en forme de griffe (7), dans lequel
la base de fixation (5) est pourvue de multiples trous de montage (51), et chacune des électrodes en forme de griffe (7) s'étend à travers le trou de montage (51) et est connectée de manière fixe à la base de fixation (5) respectivement ;
une extrémité distale du fil de traction est fixée à la base de fixation (5) ;
la base de connexion (6) est pourvue de multiples trous de guidage (61), dans lequel les ouvertures de trous de guidage adjacents (61) s'étendent dans la même direction, chacune des électrodes en forme de griffe (7) s'étend à travers le trou de guidage (61) respectivement, et les trous de guidage (61) permettent aux électrodes en forme de griffe (7) de s'étendre hors de la base de connexion (6) d'une manière dispersée ; dans lequel : chacune des électrodes en forme de griffe (7) comprend : une première section proximale (71), une deuxième section médiane (72), et une troisième section distale (73), dans lequel la deuxième section (72) est à un angle obtus par rapport à la première section (71) et pliée radialement vers l'extérieur par rapport à la première section (71), et la troisième section (73) est à un angle obtus par rapport à la deuxième section (72) et pliée radialement vers l'extérieur par rapport à la deuxième section (72), la partie de tube d'aiguille (1) comprend un premier manchon de tube (11) et un deuxième manchon de tube (12), dans lequel l'électrode centrale (3), le deuxième manchon de tube (12), la base de connexion (6) et le premier manchon de tube (11) sont connectés séquentiellement dans une direction allant d'une extrémité distale à une extrémité proximale du cathéter d'ablation par radiofréquence, une extrémité proximale de la base de connexion (6) est fixée à une extrémité distale du premier manchon de tube (11) et une extrémité distale de la base de connexion (6) est fixée à une extrémité proximale du deuxième manchon de tube (12), l'électrode centrale (3) est fixée à une extrémité distale du deuxième manchon de tube (12), la base de fixation (5) est montée de manière coulissante dans le premier manchon de tube (11), et les multiples électrodes en forme de griffe (7) sont situées à l'intérieur du premier manchon de tube (11) ; dans lequel lorsque les électrodes en forme de griffe (7) s'étendent hors de la base de connexion (6) d'une manière dispersée, les électrodes en forme de griffe (7) s'étendent et entourent l'électrode centrale (3) pour obtenir les données de température ou d'impédance autour de l'électrode centrale (3) de manière à surveiller la progression d'ablation de l'électrode centrale (3), et les extrémités distales des électrodes en forme de griffe (7) sont situées sur la même latitude, et dans lequel
la partie de poignée (2) comprend un manchon de cylindre (21) et un bouton coulissant (22), dans lequel
le bouton coulissant (22) est monté de manière coulissante sur le manchon de cylindre (21), et
une extrémité proximale du fil de traction est fixée sur le bouton coulissant (22) ; et
l'électrode centrale (3) comprend un corps d'électrode (31), un fil d'électrode, et un connecteur d'électrode, dans lequel
le corps d'électrode (31) est prévu à une extrémité distale du deuxième manchon de tube (12), une extrémité distale du fil d'électrode est connectée électriquement au corps d'électrode (31), le fil d'électrode s'étend à travers le manchon de cylindre (21), le premier manchon de tube (11) et le deuxième manchon de tube (12), une extrémité proximale du fil d'électrode est connectée électriquement au connecteur d'électrode, et le connecteur d'électrode est situé à l'extérieur du manchon de cylindre (21).

2. Cathéter d'ablation par radiofréquence selon la revendication 1, comprenant en outre un joint d'injection de liquide (24) et un tube d'injection de liquide (25), dans lequel
le tube d'injection de liquide (25) s'étend à travers le manchon de cylindre (21), la base de fixation (5) et la base de connexion (6), le corps d'électrode (31) est pourvu d'une entrée de liquide (311), et une extrémité distale du tube d'injection de liquide (25) est en communication avec l'entrée de liquide (311) ; et
le joint d'injection de liquide (24) est prévu à une extrémité proximale du tube d'injection de liquide (15), et situé à l'extérieur du manchon de cylindre (21).

3. Cathéter d'ablation par radiofréquence selon la revendication 2, dans lequel des canaux d'arrosage (312) sont prévus dans le corps d'électrode (31), dans lequel les canaux d'arrosage (312) sont en communication avec l'entrée de liquide (311), et les canaux d'arrosage (312) sont utilisés pour alimenter de manière dispersée le liquide à l'entrée de liquide (311).

4. Cathéter d'ablation par radiofréquence selon la revendication 3, dans lequel une hotte d'infiltration (32) est prévue à l'extérieur du corps d'électrode (31), dans lequel la hotte d'infiltration (32) comprend une pluralité de trous de dispersion (321) répartis dans un réseau rectangulaire, et les diamètres de trou de chaque rangée de trous de dispersion (321) diminuent dans une direction allant de l'extrémité proximale à l'extrémité distale.

5. Cathéter d'ablation par radiofréquence selon la revendication 1, dans lequel la base de connexion (6) comprend un trou d'injection de liquide (62), dans lequel le trou d'injection de liquide (62) est positionné le long de la ligne centrale de la base de connexion (6), le tube d'injection de liquide (25) s'étend à travers le trou d'injection de liquide (62), et les trous de guidage (61) sont répartis symétriquement avec le trou d'injection de liquide (62) en tant que centre ; et le trou de guidage (61) comprend une section droite (611) et une section en arc (612), dans lequel la section en arc (612) est située à une extrémité distale de la section droite (611).

6. Cathéter d'ablation par radiofréquence selon la revendication 5, dans lequel la base de connexion (6) est constituée de fibres de verre.

7. Cathéter d'ablation par radiofréquence selon la revendication 1, dans lequel les multiples électrodes en forme de griffe (7) sont des tubes creux.

8. Cathéter d'ablation par radiofréquence selon la revendication 1, dans lequel le mécanisme de détection comprend en outre une bague de fixation (4), dans lequel une extrémité distale de la bague de fixation (4) est fixée sur l'électrode en forme de griffe (7), et une extrémité proximale de la bague de fixation (4) est connectée extérieurement à un fil, permettant au fil et aux électrodes en forme de griffe (7) d'être connectés électriquement.

9. Cathéter d'ablation par radiofréquence selon la revendication 8, dans lequel la bague de fixation (4) et la base de fixation (5) sont jointes par ajustement serré pour limiter la longueur d'extension de l'électrode en forme de griffe (7).

10. Cathéter d'ablation par radiofréquence selon la revendication 1, dans lequel le second manchon de tube (12) est pourvu d'un filetage interne (121), la base de connexion (6) est pourvue d'un filetage externe (63), et le second manchon de tube (12) et la base de connexion (6) sont connectés par filetage.

11. Cathéter d'ablation par radiofréquence selon la revendication 1, dans lequel le premier manchon de tube (11) est pourvu d'un lamage (111), un trou fileté (64) est prévu à l'extrémité proximale de la base de connexion (6), et le premier manchon de tube (11) et la base de connexion (6) sont connectés par un boulon.

12. Cathéter d'ablation par radiofréquence selon la revendication 1, dans lequel un montant de montage est prévu à l'extrémité proximale de la base de connexion (6), et un ressort (8) est monté autour du montant de montage, dans lequel lorsque la base de fixation (5) se déplace vers la base de connexion (6), une extrémité distale du ressort (8) appuie contre la base de connexion (6), et une extrémité proximale du ressort (8) appuie contre la base de fixation (5).

13. Cathéter d'ablation par radiofréquence selon la revendication 1, dans lequel la première section (71) et la deuxième section (72) sont toutes deux des sections droites, et la troisième section (73) est une section en arc.

14. Cathéter d'ablation par radiofréquence selon la revendication 1, dans lequel une première rainure (53) est respectivement prévue sur les deux côtés de la base de fixation (5), un bloc de fixation (54) est prévu à l'extrémité distale du fil de traction, et le bloc de fixation (54) est inséré dans la première rainure (53), pour permettre au fil de traction et à la base de fixation (5) d'être connectés de manière fixe.

15. Système d'ablation par radiofréquence, comprenant un générateur et un cathéter d'ablation par radiofréquence selon l'une quelconque des revendications 1 à 14.
